Europäisches Patentamt

European Patent Office

Office européen des brevets

(19)

(11) **EP 0 609 117 B1**

(12) **FASCICULE DE BREVET EUROPEEN**

(45) Date de publication et mention
de la délivrance du brevet:
**07.05.1997 Bulletin 1997/19**

(51) Int Cl.6: **G01T 1/164**, G01T 1/208

(21) Numéro de dépôt: **94400127.0**

(22) Date de dépôt: **21.01.1994**

(54) **Gamma-camera à compensation de gain**

Gammakamera mit Verstärkungskompensation

Gain compensated gamma camera

(84) Etats contractants désignés:
**AT BE DE ES GB IT NL**

(30) Priorité: **27.01.1993 FR 9300805**

(43) Date de publication de la demande:
**03.08.1994 Bulletin 1994/31**

(73) Titulaire: **SOPHA MEDICAL**
**F-78534 BUC Cedex (FR)**

(72) Inventeurs:
• **Tararine, Michel**
**F-75116 Paris (FR)**

• **Benard, Jean-François**
**F-75116 Paris (FR)**

(74) Mandataire: **Lemoyne, Didier et al**
**Cabinet Ballot-Schmit**
**7, rue Le Sueur**
**75116 Paris (FR)**

(56) Documents cités:
**US-A- 3 980 886**　　　　**US-A- 4 475 042**
**US-A- 4 866 615**

EP 0 609 117 B1

## Description

La présente invention concerne une gamma-caméra comprenant des moyens de localisation de scintillations produites sous l'effet d'un rayonnement gamma dans un cristal scintillateur.

L'invention trouve une application particulièrement avantageuse dans le domaine de la médecine nucléaire pour visualiser dans un organe la répartition de molécules marquées par un isotope radioactif que l'on a injecté à un patient.

On connaît du brevet américain n° 3 011 037, par exemple, une gamma-caméra conforme au préambule comprenant un collimateur pour focaliser les photons gamma émis par le patient, un cristal scintillateur pour transformer les photons gamma en photons lumineux ou scintillations, et un réseau de tubes photomultiplicateurs qui transforment à leur tour les scintillations reçues en impulsions électriques. Ce réseau de tubes photomultiplicateurs fait partie de moyens de localisation qui, à partir des impulsions électriques fournies par les tubes, délivrent de manière connue, des signaux de coordonnées X et Y du lieu où s'est produit la scintillation, ainsi qu'un signal de validation quand l'énergie E de la scintillation appartient à une bande d'énergie prédéterminée.

Comme la scintillation est vue par plusieurs tubes photomultiplicateurs simultanément, la détermination de l'emplacement de cette scintillation sur le cristal, elle-même représentative du lieu d'émission du photon gamma d'excitation, est obtenue en calculant l'emplacement du barycentre des impulsions électriques délivrées par l'ensemble des tubes photomultiplicateurs excités par la scintillation considérée. Ce calcul est effectué simplement, selon le brevet américain précité, en reliant chaque tube photomultiplicateur, à travers une diode dite de résolution, à un circuit de pondération comprenant une matrice de résistances et des additionneurs de sortie. Les valeurs des résistances de la matrice sont fonction des positions des tubes photomultiplicateurs auxquels elles sont raccordées. La fonction de ces diodes de résolution D est de donner une réponse non-linéaire aux circuits de préamplification des signaux de sortie des tubes photomultiplicateurs, par adjonction d'un effet de seuil.

Le signal de sortie d'un tube photomultiplicateur donné ne sera transmis à travers la diode de résolution D que s'il est supérieur en valeur absolue à $S_D$-RT où :

- $S_D$ est le seuil de conduction de la diode D, par exemple 0,6 V pour une diode du type silicium,
- RT est un potentiel constant appliqué à la diode D, destiné à fixer la valeur du seuil à un niveau déterminé.

Cet effet de seuil améliore la résolution spatiale, d'où l'appellation de "seuil de résolution", et est décrit dans l'article "Improved resolution of the Anger scintillation camera through the use of threshold preamplifers " de G.H. Kerberg et N.Van Dijk, Journal of Nuclear Medecine, 13, pp. 169-171, 1972.

Par ailleurs, chacun des tubes photomultiplicateurs est relié à un circuit de linéarisation fournissant un seuil de linéarité de manière synchrone avec l'impulsion électrique issue du tube photomultiplicateur correspondant.

La fonction de ce seuil de linéarité est d'atténuer le signal de sortie du ou des tubes photomultiplicateurs fournissant le signal le plus élevé lors d'un événement. En effet, le barycentre des impulsions électriques délivrées par l'ensemble des tubes photomultiplicateurs à la suite d'une scintillation n'est qu'un estimateur de l'emplacement de cette scintillation sur le cristal. Cet estimateur comprend un élément d'erreur systématique, appelé "biais", et ce biais est la principale origine des distorsions spatiales intrinsèques de la gamma caméra.

Une des façons de minimiser ce biais est d'atténuer le signal de sortie du ou des tubes photomultiplicateurs situés en regard de la scintillation, et qui fournissent par conséquent le signal le plus élevé. En pratique, cette opération peut être facilement réalisée en dérivant le signal de sortie de chaque tube photomultiplicateur par l'intermédiaire d'une diode polarisée en inverse à l'aide d'un signal impulsionnel proportionnel à l'énergie totale de l'événement et baptisé "seuil de linéarité". Ainsi, lors d'une scintillation, les tubes photomultiplicateurs fournissant un signal d'amplitude supérieure au seuil de linéarité verront leur signal de sortie atténué par dérivation du courant à travers la diode dont la jonction devient passante.

Les tubes photomultiplicateurs présentent cependant l'inconvénient que leur gain est susceptible de dériver dans le temps par suite, notamment, de l'évolution des caractéristiques des matériaux photoémissifs ou à émission secondaire utilisés, du vieillissement des circuits électroniques de traitement, et de la sensibilité des tubes au champ magnétique. Cette variation du gain des tubes photomultiplicateurs a pour conséquence une perte notable d'information. En effet, d'une part, des impulsions électriques qui auraient normalement dû être prises en compte peuvent ne pas être validées du fait d'une énergie E de scintillation apparemment en dehors de la fenêtre d'analyse, et, d'autre part, ces variations de gain modifient les signaux utilisés pour calculer l'emplacement du barycentre de la scintillation et introduisent donc des distorsions spatiales dégradant la qualité des images obtenues.

Pour remédier à cet inconvénient, on peut avoir recours à un étalonnage préalable des tubes photomultiplicateurs. Cette procédure n'est cependant pas satisfaisante car elle prend beaucoup de temps de mise en oeuvre, de 1 à 2 heures, ne tient pas compte du vieillissement de l'électronique et n'est valable que pour une position donnée de la gamma-caméra par rapport au champ magnétique environnant.

D'autres solutions, plus avantageuses, consistent en une compensation permanente de dérive du gain des tubes photomultiplicateurs. Parmi les méthodes les plus fréquemment utilisées, on peut citer la calibration par spectrométrie nucléaire qui repose sur le fait que la position d'un pic d'absorption photoélectrique d'un spectre en énergie d'un radioisotope donné dépend du gain des tubes. Dans cette méthode, on enregistre par exemple un spectre nucléaire pendant l'acquisition clinique, le gain des tubes photomultiplicateurs étant ajusté de manière à maintenir la position du pic d'émission à l'intérieur d'une fenêtre d'énergie donnée. Cette technique de calibration connue n'est cependant pas sans limites. En effet, on observera qu'elle ne peut être utilisée avec n'importe quel radioélément: en particulier ceux qui présentent de trop nombreux pics sont à exclure. D'autre part, ce type de calibration ne peut prendre en compte la dépendance de la forme des spectres avec la configuration du patient, la diffusion des photons gammas étant différente selon la corpulence du patient. Enfin, les tubes photomultiplicateurs ne sont pas illuminés de façon homogène, de sorte que, lors de l'examen clinique où l'activité est concentrée dans une petite zone, seuls les tubes situés en face de cette zone où donc se produisent les événements sont corrigés en gain et non les autres alors qu'ils contribuent tout autant à la localisation de la scintillation. Il en résulte donc une erreur. De même, les tubes photomultiplicateurs en bord du champ ne peuvent jamais être réglés car, du fait de la présence du collimateur, ils ne reçoivent jamais de lumière directe.

Une autre méthode de calibration en gain des tubes photomultiplicateurs consiste à utiliser des sources de lumière extérieures, telles que des diodes électroluminescentes, pour illuminer chaque tube avec une quantité de lumière connue, et à en modifier le gain en conséquence lorsque la réponse de tube varie au cours du temps.

De façon pratique, les diodes électroluminescentes peuvent être placées à l'intérieur même des tubes photomultiplicateurs, ou encore à l'extérieur des tubes, une diode pouvant illuminer simultanément plusieurs tubes, par exemple trois dans le cas d'un réseau de tubes hexagonaux. Lors de l'examen clinique, les sources lumineuses émettent avec une fréquence donnée, par exemple toutes les millisecondes, des impulsions calibrées de durée déterminée, par exemple, de 1 à 2 µs. La compensation du gain est effectuée en temps réel successivement ou simultanément pour l'ensemble des tubes photomulitplicateurs après accumulation sur une durée suffisante du spectre des impulsions électriques fournies en réponse aux impulsions lumineuses reçues. Le gain des tubes peut être ajusté à l'aide d'un préamplificateur à gain variable commandé par un logiciel approprié et un convertisseur numérique/analogique.

Il faut toutefois remarquer que l'amplitude des impulsions électriques de calibration est telle qu'après sommation sur l'ensemble des tubes par les additionneurs de sortie, il se produit une saturation importante des circuits de localisation entraînant un temps de récupération relativement long, de l'ordre de 10 µs. En conséquence, la détection des événements nucléaires se trouve perturbée pendant la récupération, à moins d'augmenter le temps de blocage des circuits à 10 µs au moins, ce qui conduirait à une diminution sensible du taux de comptage.

Aussi, le problème technique à résoudre par l'objet de la présente invention est de proposer une gamma-caméra comprenant des moyens de localisation de scintillations produites sous l'effet d'un rayonnement gamma dans un cristal scintillateur, lesdits moyens de localisation comportant un réseau de tubes photomultiplicateurs contrôlés en gain à l'aide d'impulsions lumineuses calibrées, et un circuit de localisation dans lequel chaque tube photomultiplicateur est relié, d'une part, à travers une diode, dite de résolution, à un circuit de pondération comprenant un réseau de résistances connectées à des additionneurs de sortie, et, d'autre part, à un circuit de linéarisation fournissant un signal dit seuil de linéarité, gamma caméra qui permettrait d'éviter les inconvénients liés au temps de récupération de saturation des circuits, consécutif à la calibration optique des tubes, sans diminution sensible du taux de comptage des scintillations.

La solution au problème technique posé consiste, selon la présente invention, en ce que ledit circuit de linéarisation comporte des moyens pour fournir également, pendant une durée au moins égale à la durée des impulsions lumineuses calibrées, une impulsion de blocage de ladite diode de résolution vis-à-vis des impulsions électriques délivrées par le tube photomultiplicateur en réponse auxdites impulsions lumineuses.

Ainsi, ladite impulsion de blocage , constituée par une polarisation inverse de la diode de résolution, permet de réduire le courant injecté dans la matrice de résistances par l'ensemble des tubes photomultiplicateurs et d'éviter la saturation des voies de localisation. Le temps pendant lequel les événements nucléaires ne sont pas accessibles est ainsi ramené à la durée de l'impulsion de blocage, par exemple 2,5 µs au lieu de 10 µs, soit un facteur de l'ordre de 4 gagné sur la durée d'occupation du circuit de localisation consacrée à la calibration des tubes.

La description qui va suivre en regard des dessins annexés, donnés à titre d'exemples non limitatifs, fera bien comprendre en quoi consiste l'invention et comment elle peut être réalisée.

La figure 1 est un schéma partiel d'un circuit de localisation d'une gamma-caméra selon l'invention, limité à un seul tube photomultiplicateur.

La figure 2a est un chronogramme du signal de sortie du tube photomultiplicateur de la figure 1.

La figure 2b est un chronogramme du seuil de linéarité appliqué au tube de la figure 1 et correspondant au chronogramme de la figure 2a.

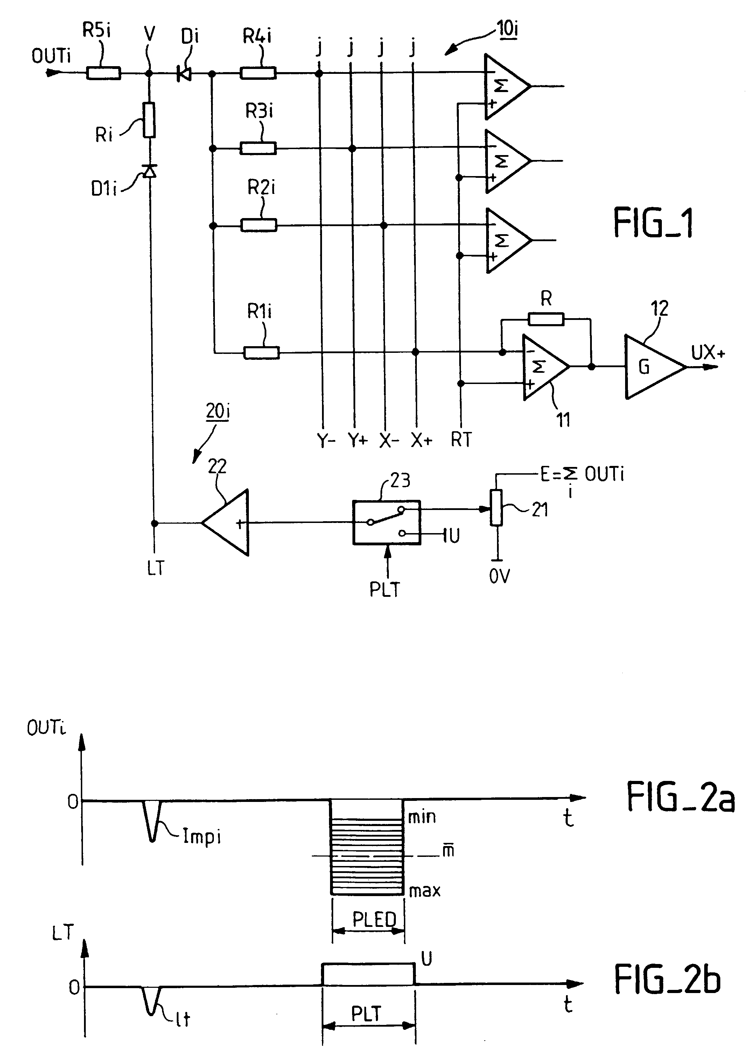

La figure 1 montre de façon schématique un circuit partiel de localisation, relatif à un tube photomultiplicateur représenté par l'indice i. Le tube i et le circuit partiel de localisation associé font partie de moyens de localisation de scintillations produites dans une gamma-caméra sous l'effet d'un rayonnement gamma dans un cristal scintillateur. Ces moyens de localisation comportent un réseau de tubes photomultiplicateurs au nombre de 58 par exemple (i=1,2...,

58), et un circuit de localisation formé de 58 circuits partiels du type de celui montré sur la figure 1.

Le circuit de la figure 1 comprend un circuit de pondération 10i auquel la sortie OUTi du tube photomultiplicateur i est reliée à travers une résistance R5i et une diode Di dite de résolution. Ce circuit de pondération comporte un réseau de résistances R1i, R2i, R3i,R4i dont les valeurs sont déterminées de manière connue pour définir les impulsions pondérées X+, X-,Y+,Y- données par:

$$X+ = \Sigma_i \ (R/R1i)\times OUTi \qquad\qquad X- = \Sigma_i \ (R/R2i)\times OUTi$$

$$Y+ = \Sigma_i \ (R/R3i)\times OUTi \qquad\qquad Y- = \Sigma_i \ (R/R4i)\times OUTi$$

Les sommations sont effectuées sur l'ensemble des tubes photomultiplicateurs de la gamma-caméra par quatre circuits additionneurs, tels que celui référencé 11 correspondant à l'impulsion pondérée X+, auxquels sont reliés tous les circuits de pondération associés à l'ensemble des tubes photomultiplicateurs, à savoir le tube i considéré ici et les autres tubes d'indice j différent de i. Les signaux issus de tous les tubes sont appliqués à la borne négative des additionneurs, tandis que leurs bornes positives sont portées à un potentiel, dit seuil de résolution RT, de 0,2 V par exemple. Les coordonnées X et Y de la scintillation détectée sont définies par:

$$X= \frac{X+ - X-}{X+ + X-} \qquad Y= \frac{Y+ - Y-}{Y+ + Y-}$$

La sortie du tube photomultiplicateur i est également reliée, toujours à travers la résistance R5i, à un circuit de linéarisation 20i dans lequel un seuil de linéarité LT, fourni par un circuit comprenant un potentiomètre 21 recevant le signal d'energie E et un amplificateur 22, est appliqué à une diode D1i, dite de linéarité, en série avec une résistance Ri.

Lorsque, comme l'indiquent les figures 2a et 2b, le tube photomultiplicateur i délivre sur sa sortie OUTi une impulsion électrique Impi en réponse à une scintillation lumineuse produite à la suite d'une émission gamma du radioélément injecté au patient, un seuil de linéarité lt est appliqué de manière synchrone à l'entrée de la diode de linéarité D1i. L'impulsion électrique Impi peut atteindre typiquement une valeur crête de -3V, tandis que le seuil de linéarité lt se situe autour de -0,2V crête.

Les tubes photomultiplicateurs de la gamma-caméra conforme à l'invention sont compensés en gain à l'aide d'impulsions lumineuses calibrées émises à une fréquence de récurrence donnée, par exemple 1 kHz, c'est-à-dire toutes les millisecondes. Ces impulsions lumineuses peuvent être fournies par des diodes électroluminescentes disposées de manière adjacente à trois tubes photomultiplicateurs hexagonaux situés en plus proches voisins. Conformément à la figure 2a, l'intensité lumineuse émise par les diodes électroluminescentes de calibration est telle que l'impulsion électrique correspondante délivrée par les tubes photomultiplicateurs peut varier entre une valeur minimale min de -2V et une valeur maximale Max de -9V avec une valeur moyenne $\overline{m}$ de -5,5V, la durée PLED de cette impulsion étant de l'ordre de 1,75 μs par exemple.

Si pendant l'application des impulsions lumineuses le seuil de linéarité LT était maintenu à 0V, la tension V en sortie de la résistance R5i, qui s'exprime par la relation générale:

$$V= (LT-0,6) + (OUTi - LT + 0,6) \times \frac{Ri}{Ri + R5i}$$

vaudrait - 1,8V avec LT=0, OUTi= $\overline{m}$ = -5,5V, Ri= 422 Ω et R5i= 1,3 kΩ. La valeur de 0,6V correspond à la tension de polarisation inverse de la diode D1i.

La tension UX+ délivrée par l'amplificateur 12 de gain G après addition par l'additionneur 11 des signaux provenant des N tubes photomultiplicateurs est donnée par :

$$UX+ = -N \times \frac{R}{R1i} \times G \times (V+0,4)$$

Avec N=58, R= 2k Ω ; R1i moyen = 15k Ω et G= 5,2, on obtient

$$UX+ = +56V$$

ce qui indique une saturation importante de l'amplificateur de sortie impliquant un temps de récupération après saturation pouvant atteindre 10µs; durée pendant laquelle des événements bien que déformés sont néanmoins pris en compte. Il en résulte des erreurs dans la localisation des scintillations et donc une perturbation de l'image fournie par la gamma-caméra.

Notons qu'en réalité la saturation serait encore plus importante car le seuil de linéarité LT est issu de la source de tension E, saturée pendant les impulsions lumineuses, et donc fortement négatif, d'où une valeur de V égale à -5,5V, les diodes Dli étant bloquées.

A l'inverse, si, pendant une durée PLT au moins égale à la durée PLED des impulsions lumineuses de calibration , 2,5 µs par exemple, on applique un seuil de linéarité LT de blocage de U= + 2V en manoeuvrant le commutateur électronique 23 à la fréquence de récurrence précitée, on trouve :

$$V = -0,29V$$

Les diodes Di de résolution sont donc bloquées et UX+=0. L'amplificateur de sortie n'est alors plus saturé, éliminant ainsi l'effet néfaste lié à la récupération après saturation.

En pratique, il y a un léger courant de conduction dans les diodes qui donne un signal UX+≤ 5V, l'amplificateur de sortie n'étant plus saturé.

Bien entendu, le raisonnement fait ici sur la coordonnée X+ est également valable pour les coordonnées X-,Y+ et Y-.

## Revendications

1. Gamma-caméra comprenant des moyens de localisation de scintillations produites sous l'effet d'un rayonnement gamma dans un cristal scintillateur, lesdits moyens de localisation comportant un réseau de tubes photomultiplicateurs contrôlés en gain à l'aide d'impulsions lumineuses calibrées, et un circuit de localisation dans lequel chaque tube photomultiplicateur (i) est relié, d'une part, à travers une diode (Di), dite de résolution, à un circuit de pondération (10i') comprenant un réseau de résistances (R1i, R2i, R3i, R4i) connectées à des additionneurs (11) de sortie, et, d'autre part, à un circuit de linéarisation (20i) fournissant un signal lt, dit seuil de linéarité, caractérisée en ce que ledit circuit de linéarisation (20i) comporte des moyens pour fournir également, pendant une durée au moins égale à la durée des impulsions lumineuses calibrées, une impulsion de blocage (U) de ladite diode de résolution (Di) vis-à-vis des impulsions électriques (Impi) délivrées par le tube photomultiplicateur (i) en réponse auxdites impulsions lumineuses.

## Patentansprüche

1. Gammakamera, umfassend Mittel zur Lokalisierung von unter der Wirkung einer Gammastrahlung in einem Szintillationskristall erzeugten Szintillationen, wobei die Mittel zur Lokalisierung ein Netz von mit Hilfe von kalibrierten Leuchtimpulsen verstärkungsgesteuerten Photomultiplikatorröhren und eine Schaltung zur Lokalisierung umfassen, in der jede Photomultiplikatorröhre (i) zum einen über eine Auflösungsdiode (Di) mit einer Gewichtungsschaltung (10i), die ein Netz von mit Ausgangsaddierern (11) verbundenen Widerständen (R1i, R2i, R3i, R4i) umfaßt, und zum anderen mit einer ein Linearitätsschwellensignal lt bereitstellenden Schaltung zur Linearisierung (20i) verbunden ist, dadurch gekennzeichnet, daß die Schaltung zur Linearisierung (20i) Mittel aufweist, die während einer Dauer, die zumindest gleich der Dauer der kalibrierten Leuchtimpulse ist, auch einen Impuls (U) zur Sperrung der Auflösungsdiode (Di) in Bezug auf von der Photomultiplikatorröhre (i) in Antwort auf die Leuchtimpulse abgegebene elektrische Impulse (Impi) bereitstellt.

## Claims

1. Gamma-camera having means for locating scintillations generated under the effect of a gamma ray in a crystal scintillator, the said locating means comprising a network of photomultiplier tubes, the gain thereof being controlled by means of calibrated light pulses, and a locating circuit in which each photomultiplier tube (i) is connected across what is known as a resolution diode (Di) and a weighting circuit (10i') having a network of resistors (Rli, R2i, R3i, R4i) connected to output adders (11) on the one hand and to a linearizer circuit (20i) emitting a signal lt, known as the linearity threshold, on the other, characterised in that the said linearizer circuit (20i) has means for supplying in addition, over a period at least equal to the duration of the calibrated light pulses, a pulse (U) from the said resolution diode (Di) to block electrical impulses (Impi) emitted by the photomultiplier tube (i) in response to the said light pulses.

FIG_1

FIG_2a

FIG_2b